# EUROPEAN PATENT APPLICATION

(11) **EP 2 290 063 A1**
(43) Date of publication of application: **02.03.2011**
(21) Application number: 08765254.1
(22) Date of filing: 06.06.2008
(51) Int. Cl.: C12N 15/09, A61K 31/7105, A61K 48/00, A61P 1/16, A61P 9/10, A61P 11/00, A61P 19/02, A61P 29/00, A61P 35/00, A61P 43/00, C07H 21/02, G01N 33/15, G01N 33/50

(54) **SIRNA OF HUMAN OSTEOPONTIN**

(71) Applicant: Gene Techno Science Co., Ltd., Sapporo-shi Hokkaido 0600002 (JP)
(72) Inventor: UEDE, Toshimitsu, Sapporo-shi Hokkaido 0040835 (JP)
(74) Representative: Herbreteau, Olivier Charles
(86) International application number: PCT/JP2008/060441
(87) International publication number: WO 2009/147742

(57) **Abstract**

The present invention provides siRNA for suppressing expression of human osteopontin in a more specific and strong manner, and a composition and a medicine comprising the same.

SEQUENCE LISTING

<110>  Gene Techno Science Co., Ltd.

<120>  siRNA of Human Osteopontin

<130>  G10-0073

<140>  PCT/JP2008/060441
<141>  2008-06-06

<160>  11

<170>  PatentIn version 3.3

<210>  1
<211>  314
<212>  PRT
<213>  Homo sapiens

<400>  1

Met Arg Ile Ala Val Ile Cys Phe Cys Leu Leu Gly Ile Thr Cys Ala
1               5                   10                  15

Ile Pro Val Lys Gln Ala Asp Ser Gly Ser Ser Glu Glu Lys Gln Leu
            20                  25                  30

Tyr Asn Lys Tyr Pro Asp Ala Val Ala Thr Trp Leu Asn Pro Asp Pro
        35                  40                  45

Ser Gln Lys Gln Asn Leu Leu Ala Pro Gln Asn Ala Val Ser Ser Glu
    50                  55                  60

Glu Thr Asn Asp Phe Lys Gln Glu Thr Leu Pro Ser Lys Ser Asn Glu
65                  70                  75                  80

Ser His Asp His Met Asp Asp Met Asp Asp Glu Asp Asp Asp Asp His
                85                  90                  95

Val Asp Ser Gln Asp Ser Ile Asp Ser Asn Asp Ser Asp Asp Val Asp

            100                 105                 110

Asp Thr Asp Asp Ser His Gln Ser Asp Glu Ser His His Ser Asp Glu
        115                 120                 125

Ser Asp Glu Leu Val Thr Asp Phe Pro Thr Asp Leu Pro Ala Thr Glu
    130                 135                 140

Val Phe Thr Pro Val Val Pro Thr Val Asp Thr Tyr Asp Gly Arg Gly
145                 150                 155                 160

Asp Ser Val Val Tyr Gly Leu Arg Ser Lys Ser Lys Lys Phe Arg Arg
                165                 170                 175

Pro Asp Ile Gln Tyr Pro Asp Ala Thr Asp Glu His Ile Thr Ser His
            180                 185                 190

Met Glu Ser Glu Glu Leu Asn Gly Ala Tyr Lys Ala Ile Pro Val Ala
        195                 200                 205

Gln Asp Leu Asn Ala Pro Ser Asp Trp Asp Ser Arg Gly Lys Asp Ser
    210                 215                 220

Tyr Glu Thr Ser Gln Leu Asp Asp Gln Ser Ala Glu Ala His Ser His
225                 230                 235                 240

Lys Gln Ser Arg Leu Tyr Lys Arg Lys Ala Asn Asp Glu Ser Asn Glu
                245                 250                 255

His Ser Asp Val Ile Asp Ser Gln Glu Leu Ser Lys Val Ser Arg Glu
            260                 265                 270

Phe His Ser His Glu Phe His Ser His Glu Asp Met Leu Val Val Asp
        275                 280                 285

Pro Lys Ser Lys Glu Glu Asp Lys His Leu Lys Phe Arg Ile Ser His
    290                 295                 300

Glu Leu Asp Ser Ala Ser Ser Glu Val Asn
305                 310

<210>  2
<211>  1447
<212>  DNA
<213>  Homo sapiens

<400>  2
gaggcagcag cagcaggagg aggcagacac agcatcgtcg ggaccagact cgtctcaggc      60

cagttgcagc cttctcagcc aaacgccgac caaggaaaac tcactaccat gagaattgca     120

gtgatttgct tttgcctcct aggcatcacc tgtgccatac cagttaaaca ggctgattct     180

ggaagttctg aggaaaagca gctttacaac aaatacccag atgctgtggc cacatggcta     240

aaccctgacc catctcagaa gcagaatctc ctagccccac agacccttcc aagtaagtcc     300

aacgaaagcc atgaccacat ggatgatatg gatgatgaag atgatgatga ccatgtggac     360

agccaggact ccattgactc gaacgactct gatgatgtag atgacactga tgattctcac     420

cagtctgatg agtctcacca ttctgatgaa tctgatgaac tggtcactga ttttcccacg     480

gacctgccag caaccgaagt tttcactcca gttgtcccca cagtagacac atatgatggc     540

cgaggtgata gtgtggttta tggactgagg tcaaaatcta agaagtttcg cagacctgac     600

atccagtacc ctgatgctac agacgaggac atcacctcac acatggaaag cgaggagttg     660

aatggtgcat acaaggccat ccccgttgcc caggacctga acgcgccttc tgattgggac     720

agccgtggga aggacagtta tgaaacgagt cagctggatg accagagtgc tgaaacccac     780

agccacaagc agtccagatt atataagcgg aaagccaatg atgagagcaa tgagcattcc     840

gatgtgattg atagtcagga actttccaaa gtcagccgtg aattccacag ccatgaattt     900

cacagccatg aagatatgct ggttgtagac cccaaaagta aggaagaaga taaacacctg     960

aaatttcgta tttctcatga attagatagt gcatcttctg aggtcaatta aaaggagaaa    1020

aaatacaatt tctcactttg catttagtca aaagaaaaaa tgctttatag caaaatgaaa    1080

gagaacatga aatgcttctt tctcagttta ttggttgaat gtgtatctat ttgagtctgg    1140

aaataactaa tgtgtttgat aattagttta gtttgtggct tcatggaaac tccctgtaaa    1200

ctaaaagctt cagggttatg tctatgttca ttctatagaa gaaatgcaaa ctatcactgt    1260

attttaatat ttgttattct ctcatgaata gaaatttatg tagaagcaaa caaaatactt    1320

ttacccactt aaaaagagaa tataacattt tatgtcacta taatcttttg ttttttaagt    1380

tagtgtatat tttgttgtga ttatcttttt gtggtgtgaa taaatctttt atcttgaatg    1440

taataag                                                              1447

<210>  3
<211>  19
<212>  DNA
<213>  Artificial

<220>
<223>  hOPN siRNA-5

<400>  3
ccaaguaagu ccaacgaaa                                                   19

<210>  4
<211>  19
<212>  DNA
<213>  Artificial

<220>
<223>  hOPN siRNA-7

<400>  4
ggucaaaauc uaagaaguu                                                   19

<210>  5
<211>  19
<212>  DNA
<213>  Artificial

<220>
<223>  hOPN siRNA-8

<400>  5
gggaaggaca guuaugaaa                                                    19


<210>  6
<211>  23
<212>  DNA
<213>  Artificial

<220>
<223>  hOPN siRNA-9

<400>  6
ttggttgaat gtgtatctat ttg                                               23


<210>  7
<211>  19
<212>  DNA
<213>  Artificial

<220>
<223>  hOPN siRNA-10

<400>  7
acuaaaagcu ucaggguua                                                    19


<210>  8
<211>  21
<212>  DNA
<213>  Artificial

<220>
<223>  hOPN siRNA-4

<400>  8
aacccugacc caucucagaa g                                                 21


<210>  9
<211>  21
<212>  DNA
<213>  Artificial

<220>
<223>  hOPN siRNA-6

<400>  9
aaguccaacg aaagccauga c                                                21

<210>  10
<211>  19
<212>  DNA
<213>  Artificial

<220>
<223>  Scr.Cont.C8

<400>  10
actctatctg cacgctgac                                                   19

<210>  11
<211>  19
<212>  DNA
<213>  Artificial

<220>
<223>  Scr.Cont.C9

<400>  11
attgtatgcg atcgcagac                                                   19

## Description

### TECHNICAL FIELD

The present invention relates to siRNA for suppressing expression of human osteopontin.

### BACKGROUND ART

Multicellular organisms are organized in hierarchical levels, i.e., cells-tissues-organs-individuals, and formed through adhesion of cells, i.e., minimum units, to each other or to extracellular materials. Tissues are composed through cell-to-cell adhesion as well as cell-to-extracellular-matrix adhesion. For such cell-to-cell adhesion, extracellular matrices, cellular adhesion molecules present in the membranes and intracellular cytoskeletons as connections between the cellular adhesion molecules play important roles. The cellular adhesion molecules involved in cell-to-extracellular-matrix adhesion assume roles of cell differentiation and signaling through adhesion of the extracellular matrices. Accordingly, analysis of the mechanism of adhesion between a cellular adhesion molecule and an extracellular matrix will lead to elucidation of mechanisms of various diseases and even to treatments thereof.

Osteopontin (OPN), one type of extracellular matrices, is a secreted acidic phosphorylated glycoprotein with a molecular weight of about 41kDa, where GRGDS sequence that is important for adhesion to integrins such as αv is present in the middle of the molecule, which is immediately followed by a thrombin cleavage site (R¹⁶⁸S¹⁶⁹). In addition, SVVYGLR sequence immediately following the GRGDS sequence binds to inflammation-related integrins α9 β 1, α4 β 1 and α4 βa 7.

OPN has been reported of its variety of functions such as cellular adhesion, cell migration, control of nitric oxide (NO) production and involvement in the immune system, and have been shown to be involved in many refractory disease conditions such as cancer metastasis, chronic inflammatory diseases such as rheumatoid arthritis and multiple sclerosis and autoimmune diseases.

In particular, direct involvement of OPN has been suggested in inflammatory diseases. Since α9 and α4 integrins as OPN receptors are expressed on neutrophils and lymphocytes, respectively, suppression of OPN functions is considered to suppress migration of leukocytes including neutrophils and expected to provide an anti-inflammatory effect.

In fact, OPN-deficient mice have been reported to show resistance to diseases such as tumor, rheumatoid arthritis, multiple sclerosis (EAE) and arterial sclerosis (Non-patent documents 1-4). Moreover, there is also a report of rheumatoid arthritis relief by the use of an OPN-neutralizing antibody (Non-patent document 5).

Thus, application of RNAi (RNA interference) technique, a novel molecule-specific knockdown technique, to treatments was examined with the expectation of its therapeutic effect through inhibition of OPN functions.

RNAi technique is a technique of rapidly suppressing expression of a specific gene at gene level using small interfering dsRNA (siRNA (small interfering RNA)) (Non-patent documents 6 and 7). This was based on the concept of providing cure of diseases by selecting multiple target sequences from mRNA sequence of OPN and synthesizing siRNAs thereof to knock down OPN.

Patent document 1 describes the use of RNA coding for an osteopontin moiety for the treatment of an IL-1β-related connective tissue disease. There is, however, no disclosure about the use of it as siRNA.

Patent document 2 describes about siRNA for FGFR (fibroblast growth factor receptors) and that FGFR enhances expression of OPN gene. There is, however, no disclosure about siRNA for OPN.

Furthermore, a kit for genetic manipulation is commercially available which comprises pools of siRNAs obtained by producing double-stranded RNAs *in vitro* with a vector and cleaving them with Dicer, a member of the RNaseIII nuclease family (SuperSilencing^{™} Human SPP1 siRNA kit (Secreted phosphoprotein 1 (osteopontin, bone sialoprotein I, early T-lymphocyte activation 1)) Non-patent document 8). This kit, however, is used for inhibiting expression of a specific gene with siRNA for the purpose of gene expression studies, and there is no disclosure about medicinal use of siRNA. Moreover, since siRNA produced according to the above-described production method also contains short RNA fragments that are not specific to the molecular, it has the drawback of low specificity for gene knockdown.

Recently, siRNAs were found from mRNAs of mouse, rat and human OPNs as siRNAs for OPN that were useful as a medicine or the like for treating diseases arising from enhancement of osteopontin (Patent document 3).
[Patent document 1] Japanese Patent Laid-Open Application No. 8-191693
[Patent document 2] US Patent Application Publication No. 2003/0143676 A1 (specification)
[Patent document 3] Japanese Patent Laid-Open Application No. 2005-323591 [Non-patent document 1] Nemoto H, Rittling SR, Yoshitake H, Furuya K, Amagasa T, Tsuji K, Nifuji A, Denhardt DT, Noda M. Osteopontin deficiency reduces experimental tumor cell metastasis to bone and soft tissues. J Bone Miner Res. 16:652-9, 2001.
[Non-patent document 2] Yumoto K, Ishijima M, Rittling SR, Tsuji K, Tsuchiya Y, Kon S, Nifuji A, Uede T, Denhardt DT, Noda M. Osteopontin deficiency protects joints against destruction in anti-type II collagen antibody-induced arthritis in mice. Proc Natl Acad Sci USA. 99:4556-4561, 2002.
[Non-patent document 3] Chabas D, Baranzini SE, Mitchell D, Bernard CC, Rittling SR, Denhardt DT, Sobel RA, Lock C, Karpuj M, Pedotti R, Heller R, Oksenberg JR, Steinman L. The influence of the proinflammatory cytokine, osteopontin, on autoimmune demyelinating disease. Science. 294:1731-5, 2001.
[Non-patent document 4] Matsui Y, Rittling SR, Okamoto H, Inobe M, Jia N, Shimizu T, Akino M, Sugawara T, Morimoto J, Kimura C, Kon S, Denhardt D, Kitabatake A, Uede T. Osteopontin Deficiency Attenuates Atherosclerosis in Female Apolipoprotein E-Deficient Mice. Arterioscler Thromb Vasc Biol. 23:1029-34, 2003.
[Non-patent document 5] Yamamoto N, Sakai F, Kon S, Morimoto J, Kimura C, Yamazaki H, Okazaki I, Seki N, Fujii T, Uede T. Essential role of the cryptic epitope SLAYGLR within osteopontin in a murine model of rheumatoid arthritis. J Clin Invest. 112:181-8, 2003.
[Non-patent document 6] Fire A, Xu S, Montgomery MK, Kostas SA, Driver SE, Mello CC. Potent and specific genetic interference by double-stranded RNA in Caenorhabditis elegans. Nature. 391:806-11, 1998.
[Non-patent document 7] Elbashir SM, Harborth J, Lendeckel W, Yalcin A, Weber K, Tuschl T. Duplexes of 21-nucleotide RNAs mediate RNA interference in cultured mammalian cells. Nature. 411:494-8, 2001.
[Non-patent document 8] Product manual for SuperSilencing™, SuperArray [as searched on March 22, 2004]. Internet < URL: http://www.superarray.com/sirnaqa.php>

### DISCLOSURE OF THE INVENTION

### Problem to be Solved by the Invention

Under such circumstances, development of siRNA has been desired that is more effective in treating human diseases caused by enhancement of human osteopontin than conventional siRNA.

### Means for Solving the Problem

The inventor of the present application has gone through keen examination, and found siRNA that suppresses expression of human osteopontin in a more specific and strong manner as compared to known siRNA, thereby accomplishing the present invention. Thus, the present invention provides the following RNA, siRNA, and a composition and a medicine comprising the same.

(1) RNA having the sequence represented by SEQ ID NO: 3, 4, 5, 6 or 7, a complementary strand thereof, or a derivative thereof.
(2) Double-stranded RNA comprising RNA having the sequence represented by SEQ ID NO: 3, 4, 5, 6 or 7 and a complementary strand thereof; or a derivative thereof.
(3) A pharmaceutical composition for suppressing expression of osteopontin, comprising the double-stranded RNA or a derivative thereof according to (2) above.
(4) A medicine for suppressing expression of osteopontin, comprising the double-stranded RNA or a derivative thereof according to (2) above as an active ingredient.
(5) A medicine for treating a disease caused by enhancement of osteopontin, comprising the double-stranded RNA or a derivative thereof according to (2) above as an active ingredient.
(6) A medicine according to (5) above, wherein the disease caused by enhancement of osteopontin is tumor, hepatitis, arterial sclerosis, multiple sclerosis, arthritis, rheumatism or pulmonary fibrosis.
(7) A siRNA expression vector comprising the RNA according to (1) above.

### EFFECTS OF THE INVENTION

siRNAs of the invention target different mRNA sites of human OPN from that targeted by conventional siRNA for human OPN mRNA, and are remarkably higher in their effects to knock down human OPN gene. Hence, they are more suitable as a medicine or the like for treating diseases caused by enhancement of human osteopontin.

### BRIEF DESCRIPTION OF THE DRAWINGS

[Figure 1] Figure 1 shows sequences targeted by human OPN siRNAs and locations thereof, along with hOPN siRNAs of the present invention (hOPN siRNA-5, 7, 8, 9, 10).
[Figure 2] Figure 2 shows sequences of human OPN siRNAs used in the example.
[Figure 3] Figure 3 is a graph showing the effects of human OPN siRNAs in suppressing OPN secretion in HT1080 cell.
[Figure 4] Figure 4 is a graph showing the effects of human OPN siRNAs in suppressing OPN secretion in NRC-12 cell.
[Figure 5] Figure 5 is a graph showing the effects of human OPN siRNAs in suppressing OPN secretion in G361 cell.

### BEST MODES FOR CARRYING OUT THE INVENTION

Hereinafter, embodiments of the invention will be described in detail.

In one embodiment, the present invention provides RNA having the sequence represented by SEQ ID NO:3, 4, 5, 6 or 7 derived from human osteopontin gene, a complementary strand thereof, or a derivative thereof. Herein, these RNAs or derivatives thereof may sometimes be referred to as "RNA of the invention".

Herein, the term "osteopontin" or "human osteopontin" refers to human osteopontin protein having the amino acid sequence represented by SEQ ID NO:1, which, also called secreted phospho protein 1, is one type of adhesion proteins included in the bone matrix. Osteopontin has an integrin-binding RGD sequence and shows an effect of adhering both osteoclasts and osteoblasts to the periphery of bone tissue. Moreover, osteopontin is negatively charged in a strong manner, which allows hydroxyapatite deposition and bone calcium retainment.

The nucleotide sequence of human osteopontin gene is represented by SEQ ID NO:2.

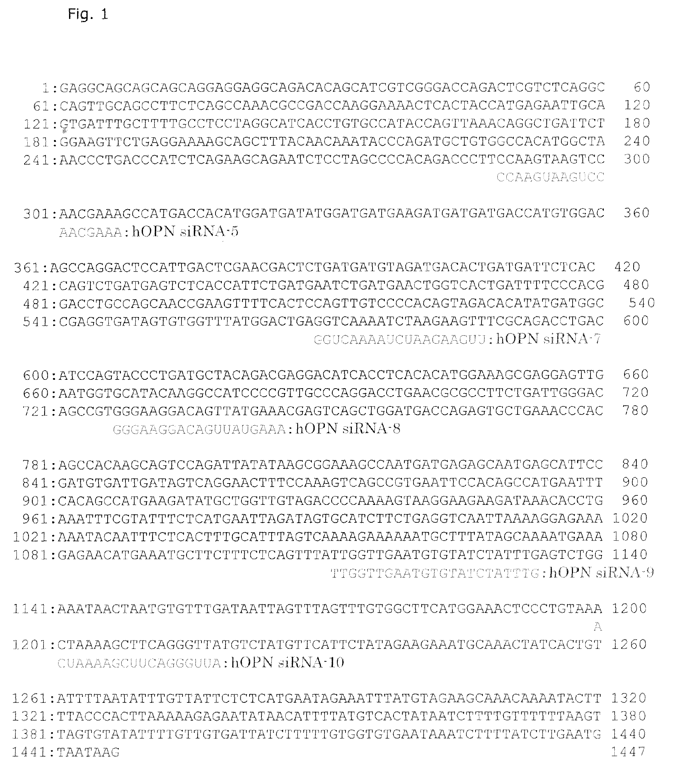

Figure 1 shows the nucleotide sequences of human osteopontin gene and sequences targeted by siRNA of the present invention.

Herein, "a complementary strand to" a certain RNA sequence refers to RNA that is complementary in its base sequence to the RNA having the certain nucleotide sequence based on base-pairing relationships, i.e., A:U and G:C. Among the complementary double-stranded DNA, a strand coding for the protein, i.e., a strand having the same sequence as mRNA, is the sense strand whereas a strand having a sequence complementary in its base sequence to the sense strand is the antisense strand.

Herein, "a derivative thereof' with respect to RNA refers to a derivative of the RNA which has been modified for enhancing its stability. Examples of such derivatives include: 3' overhang derivatives having several, preferably 2-4, more preferably 2 or 3, and most preferably two dTs added to the 3'-end; derivatives modified with polyethyleneglycol, cholesterol, 2'-o-methyl or the like; derivatives bound with two promoters such as U6 promoters, sense RNA and antisense RNA to be integrated into a so-called tandem-type siRNA expression vector, a specific example being a derivative bound with U6 promoter, sense RNA, five Ts, U6 promoter, antisense RNA and five Ts; and derivatives bound with a promoter such as U6 promoter, sense RNA, loop sequence and antisense RNA to be integrated into a so-called stem-loop-type siRNA expression vector for producing siRNA via short hairpin RNA, a specific example being a derivative bound with U6 promoter, sense RNA, a loop sequence such as gtgtgctgtcc and antisense RNA.

In another embodiment, the present invention provides RNA having the sequence represented by SEQ ID NO:3, 4, 5, 6 or 7, and double-stranded RNA comprising any of these RNAs and a complementary strand thereof, or a derivative thereof.

Herein, "siRNA (small interfering RNA)" refers to double-stranded RNA of 21-27bp that is capable of inducing RNA interference (RNAi). In addition, RNA interference refers to a phenomenon where double-stranded RNA suppresses expression of a gene having a sequence homologous to this double-stranded RNA.

Since RNAi is capable of disrupting a gene having a certain sequence, it is effective in analyzing a gene function and suppressing expression of the certain gene. It is also characterized by relatively long duration of effects for a small amount of introduction into a cell.

Double-stranded RNA comprising RNA having the human-osteopontin-gene-derived sequence represented by SEQ ID NO:3, 4, 5, 6 or 7 of the present invention and a complementary strand thereof, or a derivative thereof may be used as siRNA having such RNAi effects. Herein, these siRNAs or derivatives thereof may sometimes be referred to as "siRNA of the invention" or "human OPN siRNA of the invention".

The siRNA of the invention may be synthesized by a routine method with a commercially available DNA/RNA synthesizer, for example, Applied Biosystems 394 synthesizer.

In another embodiment, the present invention provides a siRNA expression vector carrying the RNA of the invention. The vector of the invention may be used for expressing siRNA of the invention in a target cell.

The siRNA expression vector of the invention may be prepared according to a routine method. In the case of a tandem type vector, the vector may be prepared by amplifying the promoter moiety by PCR with primers containing the sense and antisense sequences, cleaving the amplified fragment with a restriction enzyme, and inserting it downstream of the promoter of the vector (e.g., U6 promoter). In the case of a stem-loop type vector, the vector may be prepared by synthetically annealing an oligonucleotide containing sense/loop/antisense sequences, and inserting it downstream of the promoter of the vector (e.g., U6 promoter).

In another embodiment, the present invention provides a composition for suppressing the expression of osteopontin, comprising siRNA of the invention. In another embodiment, the present invention further provides a medicine for suppressing the expression of osteopontin, comprising siRNA of the invention as an active ingredient, and a medicine for treating a disease caused by enhancement of osteopontin, comprising siRNA of the invention as an active ingredient.

Furthermore, a composition comprising siRNA of the invention may be prepared as a pharmaceutical composition by itself or as a mixture with known pharmaceutically acceptable carriers (including an excipient, a filler, a binder, a lubricant and the like) and commonly used additives, for usage in treatment of human diseases. Since the siRNA of the invention is effective in suppressing expression of osteopontin, a pharmaceutical composition comprising the siRNA of the invention as an active ingredient may be used for treating diseases caused by enhancement of osteopontin expression, typically, tumor, hepatitis, arterial sclerosis, multiple sclerosis, arthritis, rheumatism, or pulmonary fibrosis. For *in vivo* stability and a method for delivery to body, a pharmaceutical composition of the invention may employ techniques for binding to a delivery carrier such as liposome, a site-specific antibody or a cell-species-specific functional peptide. In addition, it may be orally or parenterally administered according to the prepared formulation (orally-administered agents such as tablet, pill, capsule, powder, granule and syrup; and parenterally-administered agents such as injectable agent, topical agent and suppository) or the like. The dosage varies depending on the type of the active ingredient, administration route, administration target, or age, weight and conditions of the patient and thus cannot simply be defined, but in general, a daily dose of several mg to about 2 g, preferably about several tens mg may be administered once or in several times a day.

Hereinafter, the present invention will be described by means of examples, which should not limit the scope of the present invention.

### EXAMPLES

### (Preparation of oligonucleotide)

Libonucleoside 3'-phosphoramidite (GLEN Research) was used to synthesize an oligoribonucleotide with an automatic DNA synthesizer (Applied Biosystem Model 394A). Each RNA fragment was synthesized on a scale of 1 µmol. At the end of synthesis, CPG (Controlled Pore Glass) bound with the synthesized oligonucleotide was treated with a concentrated ammonia water: ethanol (3: 1 v/v) mixture at room temperature for 2 hours to excise the oligoribonucleotide from the CPG resin, which was further heated at 55°C for 16 hours. hOPN siRNA-4 and hOPN siRNA-6 were synthesized as positive controls while scrambled sequences Scr. Cont. C8 and Scr. Cont. C9 with GC contents of 52% and 47%, respectively, and having the following structures that differ from any of the siRNA sequences were synthesized as negative controls.
Scr. Cont. C8 : 5'- ACTCTATCTGCACGCTGAC -3' (SEQ ID NO:10)
Scr. Cont. C9 : 5'- ATTGTATGCGATCGCAGAC -3' (SEQ ID NO:11)

The solvent was distilled away, and 1ml of 1M TBAF (tetrabutylammonium fluoride)/THF (tetrahydrofuran) solution was added to the residue and agitated at 37°C for 16 hours. To this, 5 ml of 0.1M triethylammonium acetate (pH 7.0) was added and the resultant was separated by C18 (Waters) open column chromatography (column size 1.5 x 12 cm: eluted with a gradient of solvents of 5-40% acetonitrile in 50 mM aqueous triethylammonium bicarbonate solution). Fractions that presented color development of dimethoxytrityl eluted with acetonitrile with a concentration of about 30% were collected, to which 5 ml of 0.01N hydrochloric acid was added, followed by agitation for 15 minutes to remove the dimethoxytrityl group. The resultant was neutralized with 0.1N ammonia water and the aqueous layer was washed with ethyl acetate. The resultant was dissolved into 1 ml of sterilized water after distilling the solvent away. Oligoribonucleotides in these fractions were separated and collected by reversed-phase HPLC, and further separated and collected by ion-exchange HPLC and purified. The resultant oligonucleotides were subjected to the experiment described below.

### (Conditions for reversed-phase and ion-exchange HPLCs)

### Reversed-phase HPLC

- Column:: µ-Bondasphere (C-18) column, Φ3.9 x 150 mm (Waters)
- Solvents:: Solution A 5% acetonitrile/0.1M TEAA (pH7.0); and
Solution B 25% acetonitrile/0.1M TEAA (pH7.0).

### Ion-exchange HPLC

- Column:: TSK gel DEAE 2SW column, 4.6 x 250 mm, Tosoh
- Solvent:: Solution A 20% acetonitrile; and
Solution B 20% acetonitrile in 2M ammonium formate.

### (Method for introducing OPN siRNA into cultured cell)

According to a lipofection technique using lipofectamine 2000 (Invitrogen, Carlsbad, CA), OPN siRNA was introduced into cultured cells proliferated in a 24-well plate at logarithmic growth phase. As the cultured cells, HT1080, NRC-12 and G361 cells that were endogenously expressing OPN were used. Here, HT1080, NRC-12 and G361 cells were fibrosarcoma cell, kidney cancer cell and melanoma cell, respectively.

To 50 µL of Opti-MEM I medium (Invitrogen), 2 µL of lipofectamine 2000 was added and left for 5 minutes. In another tube, 0.8 µg of siRNA was added to 50 µL of TIL medium. The contents of both tubes were mixed together and left for 20 minutes to prepare a siRNA-lipofectamine 2000 complex. The cells were washed with TIL medium, added with 500 µL of TIL medium, further added with the siRNA-lipofectamine 2000 complex, and cultured for 24 hours. Subsequently, the supernatant was collected to determine the OPN expression level by ELISA.

### (ELISA)

RNAi effects of human OPN siRNAs were examined in terms of suppression of OPN secretion by measuring concentrations of OPN secreted from the cells with human osteopontin measurement ELISA kit (Immuno-Biological Laboratories).

### (Therapeutic effects of OPN siRNAs)

The abilities of the human OPN siRNAs of the present invention to suppress metastasis in HT1080 (Human fibrosarcoma), NRC-12 and G361 cells were examined using the above-described ELISA kit. Sense strands of human OPN siRNAs used are shown in Table 1 below (dT at the 3' end being omitted).

**[Table 1]**

| Name of sequence | Sequence | SEQ ID NO: |
|---|---|---|
| hOPN siRNA-5 | 5'-CCAAGUAAGUCCAACGAAA-3' | 3 |
| hOPN siRNA-7 | 5'-GGUCAAAAUCUAAGAAGUU-3' | 4 |
| hOPN siRNA-8 | 5'-GGGAAGGACAGUUAUGAAA-3' | 5 |
| hOPN siRNA-9 | 5'-TTGGTTGAATGTGTATCTATTTG-3' | 6 |
| hOPN siRNA-10 | 5'-ACUAAAAGCUUCAGGGUUA-3' | 7 |
| hOPN siRNA-4 | 5'-AACCCUGACCCAUCUCAGAAG-3' | 8 |
| hOPN siRNA-6 | 5'-AAGUCCAACGAAAGCCAUGAC-3' | 9 |

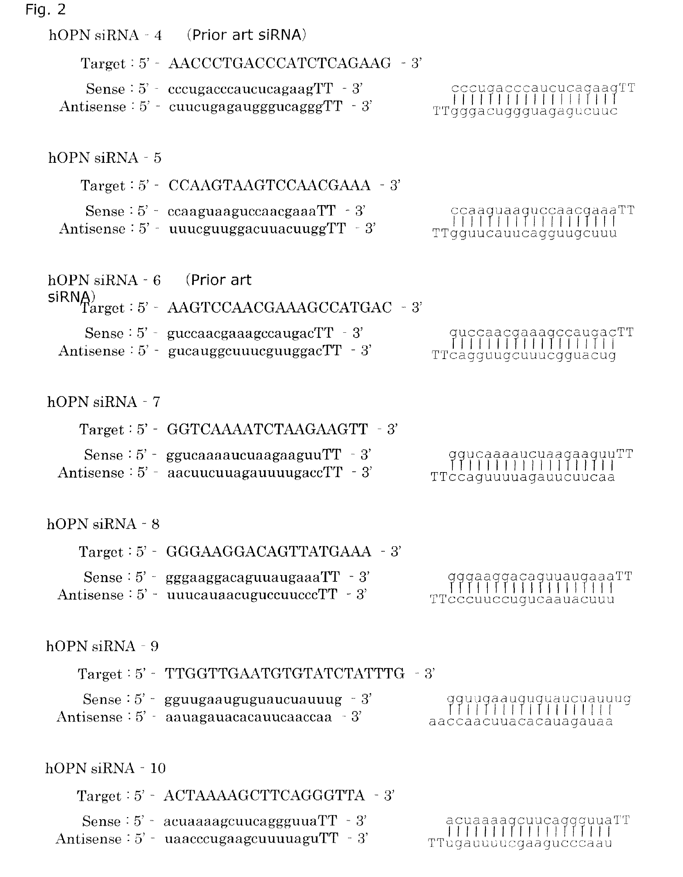

The sequences of siRNAs used in this example are shown in more detail in Figure 2.

As controls, scrambled sequence Scr. Cont. C8 with a GC content of 52% and scrambled sequence Scr. Cont. C9 with a GC content of 47% which were different from any of the siRNA sequences, as well as Lipo. Cont. consisting only of lipofectamine 2000 were used.

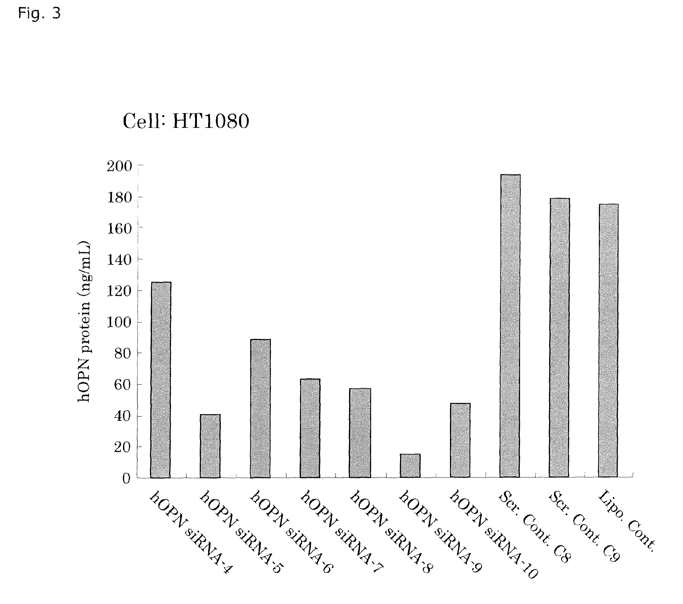

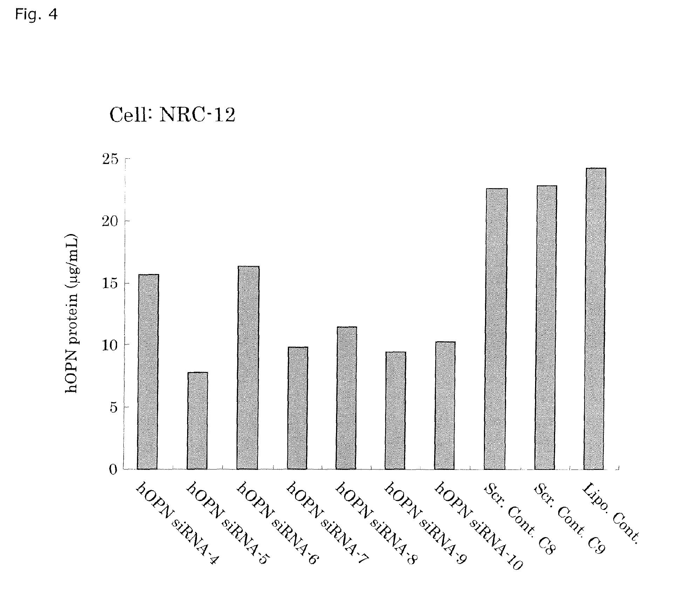

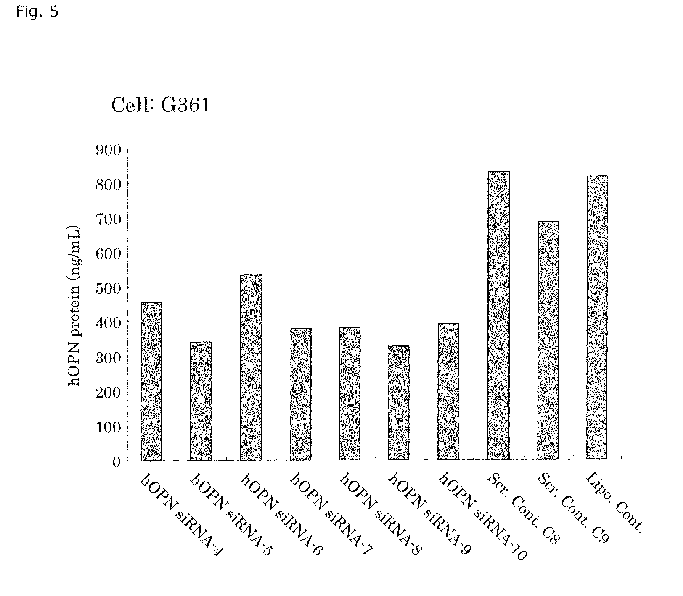

The results from these experiments are shown in Figures 3-5. The human OPN siRNAs of the invention (hOPN siRNAs-5, 7, 8, 9 and 10) showed remarkably higher OPN knockdown effect than prior art human OPN siRNAs (hOPN siRNAs-4 and 6) in all of the cell lines, i.e., HT1080 (Figure 3), NRC-12 (Figure 4) and G361 (Figure 5).

### INDUSTRIAL APPLICATION

Thus, since human OPN siRNA of the present invention shows significantly higher suppression effect on OPN secretion than prior art OPN siRNA, it may advantageously be used as a medicine for treating a disease caused by enhancement of human osteopontin.

## Claims

1. RNA having the sequence represented by SEQ ID NO:3, 4, 5, 6 or 7, a complementary strand thereof, or a derivative thereof.

2. Double-stranded RNA comprising RNA having the sequence represented by SEQ ID NO:3, 4, 5, 6 or 7 and a complementary strand thereof; or a derivative thereof.

3. A pharmaceutical composition for suppressing expression of osteopontin, comprising the double-stranded RNA or a derivative thereof according to Claim 2.

4. A medicine for suppressing expression of osteopontin, comprising the double-stranded RNA or a derivative thereof according to Claim 2 as an active ingredient.

5. A medicine for treating a disease caused by enhancement of osteopontin, comprising the double-stranded RNA or a derivative thereof according to Claim 2 as an active ingredient.

6. A medicine according to Claim 5, wherein the disease caused by enhancement of osteopontin is tumor, hepatitis, arterial sclerosis, multiple sclerosis, arthritis, rheumatism or pulmonary fibrosis.

7. A siRNA expression vector comprising the RNA according to Claim 1.
